# FASCICULE DE BREVET EUROPEEN

(11) **EP 1 490 092 B1**
(45) Date de publication et mention de la délivrance du brevet: **16.05.2007**
(21) Numéro de dépôt: 03744398.3
(22) Date de dépôt: 14.03.2003
(51) Int. Cl.: A61K 38/10, A61P 17/00, A61K 8/64

(54) **COMPOSITION COSMETIQUE OU PHARMACEUTIQUE COMPRENANT DES PEPTIDES**
PEPTIDHALTIGE KOSMETISCHE ODER PHARMAZEUTISCHE ZUSAMMENSETZUNG
COSMETIC OR PHARMACEUTICAL COMPOSITION CONTAINING PEPTIDES

(30) Priorité: 18.03.2002 FR 0203306
(43) Date de publication de la demande: 29.12.2004
(73) Titulaire: Société d'Extraction des Principes Actifs ( Vincience SA), 06410 Biot (FR)
(72) Inventeur: DAL FARRA, Claude, F-06650 Opio (FR); DOMLOGE, Nouha, F-06560 Valbonne (FR)
(86) Numéro de dépôt international: PCT/FR2003/000817
(87) Numéro de publication internationale: WO 2003/077936

(56) Documents cités:
- WO-A-01/83516
- WO-A-99/42126
- US-A- 5 696 229
- US-B1- 6 245 342
- PATENT ABSTRACTS OF JAPAN vol. 011, no. 085 (C-410), 14 mars 1987 (1987-03-14) & JP 61 238712 A (SHISEIDO CO LTD), 24 octobre 1986 (1986-10-24)
- C. DAL FARRA ET AL.: "Development of a new fibronectin peptide, and its effect on epidermal cell adhesion." MOLECULAR BIOLOGY OF THE CELL, vol. 12, no. supplement, novembre 2001 (2001-11), page 190a XP008012370
- C. DAL FARRA ET AL.: "New laminin-peptide promotes extracellular matrix production and modulates cell adhesion." JOURNAL OF INVESTIGATIVE DERMATOLOGY, vol. 119, no. 1, juillet 2002 (2002-07), page 231 XP008012371 Malden, MA, US

## Description

La présente invention concerne le domaine de la pharmaceutique, et plus particulièrement le domaine de la dermatologie ainsi que celui de la cosmétique.

La présente invention a pour objet une composition cosmétique et/ou dermatologique et/ou pharmaceutique comprenant, comme principe actif, au moins un peptide de séquence
Arg-Asp-Phe-Thr-Lys-Ala-Thr-Asn-Ile-Arg-Leu-Arg-Phe-Leu-Arg

L'invention comprend aussi son utilisation afin de traiter, entre autres, les manifestations du vieillissement cutané.

La peau est un organe de revêtement recouvrant la totalité de la surface du corps. C'est un organe vital assurant des fonctions multiples telles que des fonctions sensitives, protectrices vis à vis d'agressions externes multiples, immunitaires, métaboliques ou encore thermorégulatrices. Ces rôles sont rendus possibles grâce à une structure complexe qui associe des structures tissulaires variées.

La peau, comme tous les autres organes, est soumise au vieillissement. La première manifestation générale du vieillissement cutané est l'apparition de ridules. La peau devient moins souple, plus fine, souvent sèche et elle perd son élasticité. La perte de souplesse, de fermeté et d'hydratation de la peau, ainsi que l'apparition des rides et des ridules sont dues, vraisemblablement, à la perte inégale des différents types de macromolécules de la matrice cutanée.

La jonction dermo-épidermique est une structure complexe qui représente une zone d'interface entre le derme et l'épiderme. Elle se compose du pôle basal des kératinocytes ainsi que de la lame basale, une structure spécialisée de la matrice extra-cellulaire.

La synthèse de la lame basale est précocement altérée lors du vieillissement, on observe ainsi un ralentissement de la synthèse des molécules qui la compose ainsi qu'une augmentation de l'activité des enzymes qui la dégrade. L'adhésion cellulaire et la communication cellulaire entre le derme et l'épiderme diminuent donc de la même manière *(Gilchrest BA. Skin aging and photoaging : an overview. J Am Acad Dermatol. 21: 610-3, 1989*.).

La lame basale est constituée de macromolécules qui jouent un rôle primordial notamment dans les phénomènes d'ancrage et de différenciation cellulaire ; parmi les protéines qui la composent, on peut citer notamment la laminine, le collagène, la fibronectine ou encore l'élastine.

Elément clé de la matrice extra-cellulaire, la laminine participe à l'adhésion cellulaire et au ciment inter-cellulaire. Pour assurer ces fonctions d'adhésion, les laminines se lient à des récepteurs trans-membranaires tels que des intégrines. Cette interaction initie, de la même manière, la communication cellulaire, la différenciation cellulaire, la migration ou encore la prolifération cellulaire.

Les professionnels de la santé et de la cosmétique recherchent depuis de nombreuses années des moyens pour lutter ou, tout au moins, pour réduire les phénomènes du vieillissement cutané, ainsi que des moyens afin d'augmenter la résistance de la peau aux agressions extérieures et au stress qu'elle subit quotidiennement. Un certain nombre de substances introduites dans des produits cosmétiques ou pharmaceutiques ont vu le jour, mais il reste encore des progrès à faire afin de pouvoir disposer de produits cosmétiques ou pharmaceutiques capables de régler ces problèmes de manière satisfaisante.

Le problème technique à résoudre a donc été, pour les inventeurs, de trouver une nouvelle substance, cosmétiquement ou pharmaceutiquement acceptable, qui soit capable de combattre le phénomène de vieillissement cutané et, notamment, d'agir au niveau du phénomène d'adhésion cellulaire.

Or, les inventeurs ont réussi à sélectionner des substances particulières présentant des propriétés remarquables lorsque celles-ci sont appliquées sur la peau, notamment des propriétés au niveau de la matrice extra-cellulaire, et plus particulièrement au niveau de la lame basale.

De manière inattendue, les inventeurs ont découvert que les peptides correspondant à la formule générale : Arg-Asp-Phe-Thr-Lys-Ala-Thr-Asn-Ile-Arg-Leu-Arg-Phe-Leu-Arg, ont des propriétés remarquables et, notamment, qu'ils aident à prévenir les phénomènes du vieillissement cutané. La demanderesse a ainsi découvert un produit qui a un effet sur la production de protéines essentielles à la peau, notamment des protéines de la matrice extra-cellulaire.

A la connaissance de la demanderesse, il n'a jamais été décrit dans l'art antérieur l'utilisation d'un peptide de séquence Arg-Asp-Phe-Thr-Lys-Ala-Thr-Asn-Ile-Arg-Leu-Arg-Phe-Leu-Arg en cosmétique et/ou en dermatologie et/ou en pharmaceutique.

Ainsi, selon un premier aspect, la présente invention a pour objet une composition cosmétique et/ou dermatologique et/ou pharmaceutique caractérisée par le fait qu'elle contient, comme principe actif, une quantité efficace d'au moins un peptide de formule (I) :
(I) Arg-Asp-Phe-Thr-Lys-Ala-Thr-Asn-Ile-Arg-Leu-Arg-Phe-Leu-Arg.

Il se peut que pour des questions de résistance à la dégradation, il soit nécessaire d'utiliser une forme protégée du peptide. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées, elles sont bien connues de l'homme du métier comme, par exemple, l'acylation ou l'acétylation de l'extrémité amino-terminale, ou l'amidation ou l'estérification de l'extrémité carboxy-terminale.

Ainsi, l'invention concerne une utilisation du peptide, tel que défini précédemment, caractérisée par le fait que le peptide est sous forme protégée ou non.

De préférence, on utilise une protection basée soit sur l'acylation ou l'acétylation de l'extrémité amino-terminale, soit sur l'amidation ou l'estérification de l'extrémité carboxy-terminale, soit encore des deux.

Dans le domaine des acides aminés, la géométrie des molécules est telle qu'elles peuvent théoriquement se présenter sous la forme d'isomères optiques différents. Il existe en effet une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). La nature n'a retenu pour les acides aminés naturels que la conformation lévogyre. En conséquence, un peptide d'origine naturelle ne sera constitué que d'acides aminés de type L-aa.

Cependant la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre.

Ainsi, les acides aminés constituant le peptide selon l'invention, peuvent être sous configuration L- et D-, de manière préférentielle, les acides aminés sont sous forme L-. Le peptide selon l'invention peut donc être sous forme L-, D- ou DL-.

Les peptides, objet du présent brevet, peuvent être obtenus soit par synthèse chimique classique (en phase solide ou en phase homogène liquide), soit par synthèse enzymatique (*Kullman et al., J. Biol. Chem. 1980, 225, 8234*) à partir d'acides aminés constitutifs ou de leurs dérivés.

Les peptides selon l'invention peuvent être aussi obtenus par fermentation d'une souche de bactéries modifiées ou non par génie génétique pour produire les peptides de séquence indiquée précédemment et leurs fragments, ou encore par extraction de protéines d'origine animale ou végétale, suivie d'une hydrolyse contrôlée qui libère les fragments peptidiques de taille moyenne et de petite taille dont il est question.

Il est possible, mais non nécessaire pour réaliser l'invention, d'extraire soit les protéines concernées d'abord et de l'hydrolyser ensuite, soit d'effectuer l'hydrolyse d'abord sur un extrait brut et de purifier les fragments peptidiques ensuite.

D'autres procédés plus simples ou plus complexes peuvent être envisagés par l'homme du métier connaissant le métier de synthèse, d'extraction et de purification des protéines et des peptides.

Ainsi le peptide selon l'invention peut être un peptide d'origine naturelle ou synthétique. Préférentiellement selon l'invention, le peptide est obtenu par synthèse chimique.

Par ailleurs, il est bien entendu que l'invention concerne aussi toutes protéines ou fragments de protéines contenant le peptide de séquence : Arg-Asp-Phe-Thr-Lys-Ala-Thr-Asn-Ile-Arg-Leu-Arg-Phe-Leu-Arg. Lorsque l'on utilise un polypeptide contenant ledit peptide, il est bien entendu que celui-ci est choisi de telle sorte que les acides aminés l'entourant, tant par leur nature que par la structure secondaire du peptide qu'ils vont induire, n'empêchent pas celui-ci d'exercer l'activité pour laquelle il est utilisé dans la présente invention.

La composition selon l'invention peut être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage.

La composition selon l'invention est préférentiellement une composition cosmétique et/ou dermatologique adaptée à l'administration par voie topique cutanée comprenant un milieu cosmétiquement ou pharmaceutiquement acceptable.

Il est bien évident que l'invention s'adresse aux mammifères en général et plus particulièrement aux êtres humains.

La quantité efficace de principe actif correspond à la quantité nécessaire pour obtenir le résultat désiré.

Selon un mode de réalisation avantageux de l'invention, le peptide précité est présent dans les compositions de l'invention à une concentration représentant de 0,005 à 500 ppm (p/p) environ, et préférentiellement à une concentration représentant de 0,1 à 50 ppm (p/p) environ.

Selon un mode de réalisation avantageux de l'invention, le peptide précité est préalablement solubilisé dans un ou plusieurs solvants cosmétiquement ou pharmaceutiquement acceptable comme l'eau, l'éthanol, le propanol ou l'isopropanol, le propylène glycol, le butylène glycol, le dipropylène glycol, les diglycols éthoxylés ou propoxylés, les polyols cycliques ou tout mélange de ces solvants.

Selon encore un autre mode de réalisation avantageux de l'invention, les peptides précités sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées.

Préférentiellement, les compositions selon la présente invention se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les poils ou les cheveux.

Ces compositions pourront notamment se présenter sous forme d'une solution aqueuse, hydralcoolique ou huileuse ; d'une émulsion huile-dans-eau, eau-dans-huile ou émulsions multiples ; elles aussi peuvent se présenter sous forme de crèmes, de suspensions, ou encore de poudres, adaptées à une application sur la peau, les muqueuses, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Pour l'injection, la composition selon l'invention peut se présenter sous forme de lotion aqueuse, huileuse ou sous forme de sérum. Pour l'application sur les yeux, la composition peut se présenter sous forme de gouttes. Pour l'ingestion, elle peut se présenter sous forme de capsules, de granulés, de sirops ou de comprimés.

Ces compositions comprennent, en outre, tout additif usuellement utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc...

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition.

Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme compositions cosmétiques ou pharmaceutiques pour la peau, les muqueuses et/ou les semi-muqueuses.

Elles trouvent une application toute particulière en tant que produit de protection et/ou de soin de la peau, ou encore en tant que composition anti-rides et/ou anti-âge.

On peut également envisager une application dans le domaine des compositions de maquillage de la peau du visage et du corps, telles que les rouges à lèvres, les fond de teint, les crèmes teintées, les sticks anti-cernes, ou les compositions anti-solaire ou de bronzage artificiel.

Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps.

La composition selon l'invention peut également consister en des préparations solides comprenant également des savons ou des pains de nettoyage.

La composition peut être aussi conditionnée sous forme d'une composition pour aérosol comprenant également un agent propulseur sous pression.

La composition peut être aussi à usage bucco-dentaire, par exemple une pâte de dentifrice.

La composition de l'invention peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule, ou encore d'un aliment ou complément nutritionnel.

Selon l'invention, on peut, entre autres, ajouter à la composition de l'invention d'autres agents actifs destinés notamment à la prévention et/ou au traitement des manifestations cutanées du vieillissement et/ou à la protection de la peau contre les agressions extérieures.

L'invention a également pour objet l'utilisation, comme ingrédient actif, d'au moins un peptide de séquence Arg-Asp-Phe-Thr-Lys-Ala-Thr-Asn-Ile-Arg-Leu-Arg-Phe-Leu-Arg; dans ou pour la préparation d'une composition cosmétique et/ou dermatologique et/ou pharmaceutique.

Il a été constaté que le peptide selon l'invention possède de nombreuses actions au niveau de la peau, notamment qu'il permet de lutter contre les phénomènes du vieillissement cutané et qu'il permet de protéger la peau contre tous types d'agressions extérieures.

Par ailleurs, ledit agent actif augmente l'expression des protéines de la peau et/ou améliore leur stabilité. Les peptides selon l'invention favorisent ainsi la régénération tissulaire, en augmentant le phénomène de différentiation cellulaire, et il renforce donc la fonction barrière de la peau.

L'invention a donc pour autre objet l'utilisation d'au moins un peptide de formule (I) tel que défini précédemment, ou d'une composition cosmétique le comprenant, afin de lutter de manière curative et/ou préventive contre les phénomènes du vieillissement cutané, mais aussi afin d'améliorer l'aspect de la peau.

Par l'expression "améliorer l'aspect de la peau", on entend tous les phénomènes qui sont susceptibles d'avoir pour conséquences une amélioration visuelle de l'état de la peau. La peau présentera une meilleure apparence ; elle sera, par exemple, beaucoup plus belle, ferme et/ou lisse. Toutes les petites imperfections seront diminuées ou supprimées. L'aspect papyracé de la peau sera, par exemple, atténué.

Par modifications cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultra-violets.

Il a été mis en évidence de nombreuses propriétés de la composition selon l'invention. En effet, telles que les expériences le démontrent dans les exemples, le peptide de formule (I) de séquence Arg-Asp-Phe-Thr-Lys-Ala-Thr-Asn-Ile-Arg-Leu-Arg-Phe-Leu-Arg, ont des effets bénéfiques sur la synthèse des protéines de la matrice extra-cellulaire.

L'invention a donc pour autre objet l'utilisation d'au moins un peptide tel que défini précédemment, ou d'une composition cosmétique le comprenant, afin d'augmenter l'expression des protéines de la matrice extra-cellulaire.

On peut citer, à titre d'exemple de protéines de la matrice extra-cellulaire, des protéines telles que le collagène, la fibronectine ou encore la laminine. Toutes ces protéines sont constitutives de la matrice et y occupent un rôle fondamental ; elles jouent, entre autres, un rôle important dans l'adhésion cellulaire.

Plus particulièrement, le peptide ou la composition selon l'invention permettent d'augmenter l'expression de laminine, notamment, ils permettent d'augmenter la synthèse de la laminine-5. Cette protéine interagit avec les intégrines du pôle basal des kératinocytes, permettant ainsi d'ancrer les cellules sur le réseau bidimensionnel de la jonction dermo-épidermique.

Selon un autre aspect, le peptide ou la composition selon l'invention permettent d'augmenter la synthèse des intégrines, notamment l'expression des sous-unités α6 et β1.

L'adhésion cellulaire s'effectue, notamment, grâce à ces glycoprotéines membranaires et en s'associant à diverses molécules de la matrice extra-cellulaire comme la fibronectine ou la laminine. Les intégrines sont très impliquées dans l'adhésion des kératinocytes à la matrice extra-cellulaire, dans les liaisons cellule-cellule et dans la cohésion de l'assise basale de l'épiderme.

Le peptide selon l'invention, en induisant une augmentation de l'expression des intégrines, va donc induire une augmentation de la capacité d'adhérence des cellules cutanées.

Ainsi, l'invention a pour autre objet l'utilisation dans ou pour la préparation d'une composition cosmétique ou dermatologique, d'une quantité efficace de peptides de formule (I); les peptides ou la composition étant destinés à augmenter l'adhésion cellulaire. Plus particulièrement, les peptides ou la composition sont destinés à augmenter l'adhésion des cellules cutanées.

Par le terme "adhésion cellulaire" on entend d'une part l'adhésion entre les cellules et la matrice extra-cellulaire et, d'autre part, l'adhésion des cellules entre elles. Par le terme "adhésion des les cellules cutanées", on entend d'une part l'adhésion entre les cellules cutanées et la matrice extra-cellulaire et, d'autre part, l'adhésion des cellules cutanées entre elles.

Par ailleurs, la dynamique d'adhésion cellulaire est liée aux propriétés mécaniques de la matrice extra-cellulaire. La création d'une matrice extra-cellulaire très riche en protéines qui la composent, notamment riche en fibronectine, facilite le phénomène de re-épithélisation. La re-épithélisation est le terme utilisé pour décrire le renouvellement de la peau par un nouvel épithélium de kératinocytes. Ce processus fait intervenir des mécanismes biologiques complexes et nécessite la présence de molécules d'adhésions ainsi que la présence d'une matrice extra-cellulaire. Le peptide selon l'invention facilite donc le processus de re-épithélisation et de cicatrisation de la peau.

Un autre aspect de l'invention est donc l'utilisation dans ou pour la préparation d'une composition, d'une quantité efficace des peptides tels que définis précédemment ; les peptides ou la composition étant destinés à accélérer la cicatrisation et la régénération tissulaire. La composition selon l'invention est donc utilisée pour la régénération tissulaire de la peau et/ou des phanères en général.

Le peptide et la composition, selon l'invention, présente ainsi des propriétés intéressantes vis-à-vis de la peau et/ou des phanères, notamment des propriétés de régénération tissulaire qui permettent, par exemple, une cicatrisation améliorée ainsi que de propriétés anti-âge permettant de prévenir et/ou de réduire les effets liés au vieillissement de la peau.

Cette composition permet la restructuration de l'épiderme, et contribue à une protection des couches les plus profondes. L'épiderme devient plus épais, plus profond et est en continuelle interaction avec le derme.

Ainsi, le peptide selon l'invention renforce la fonction barrière de la peau et, il permet notamment de protéger la peau et/ou les cheveux d'une manière efficace contre tous types d'agressions extérieures.

On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums.

L'invention peut être mise en oeuvre notamment en appliquant les compositions cosmétiques telles que définies ci-dessus, selon la technique d'utilisation habituelle de ces compositions comme, par exemple, l'application de crèmes, de gels, de sérums, de lotions, de laits, de shampooings ou de compositions anti-solaires, sur la peau ou sur les cheveux, ou encore, l'application de dentifrice sur les gencives.

D'autres avantages et caractéristiques de l'invention apparaîtront mieux à la lecture des exemples donnés à titre illustratif et non limitatif.

### Exemple 1 - Etude de la stabilité du peptide Arg-Asp-Phe-Thr-Lys-Ala-Thr-Asn-Ile-Arg-Leu-Arg-Phe-Leu-Arg.

Le peptide Arg-Asp-Phe-Thr-Lys-Ala-Thr-Asn-Ile-Arg-Leu-Arg-Phe-Leu-Arg a été analysé à une concentration de 10⁻⁴ M par HPLC sur une colonne de type C18, et avec un gradient linéaire eau/TFA 0,1 % - acétonitrile/TFA 0,1 %. Après 24 heures à différentes températures (25 °C, 37 °C et 60°C), aucune dégradation du peptide n'a été observée. De plus, après 8 jours à 25 °C, le peptide ne présente toujours pas de dégradation.

Des fibroblastes et des kératinocytes humains ont été cultivés pendant 24 heures à 37°C et à 5 % de CO2. Pendant cette période, les cellules vont libérer, dans le milieu de culture, de nombreuses enzymes de dégradation. Par la suite, le milieu de culture est retiré pour être mis en présence du peptide. Les résultats des analyses effectuées démontrent qu'après 24 heures le peptide ne présente quasiment pas de dégradation.

Un test est effectué en appliquant le peptide sur des fibroblastes ainsi que sur des kératinocytes humains en culture. Une analyse HPLC du milieu de culture révèle que la concentration en peptide décroît rapidement, c'est-à-dire en quelques heures.

Ces résultats, pris dans leur ensemble, suggèrent une très bonne stabilité du peptide dans le temps ainsi que la possibilité d'une pénétration du peptide dans la cellule. Cette hypothèse est ensuite confortée par les tests d'efficacité exposés dans les exemples suivants.

### Exemple 2 - Mise en évidence de l'effet du peptide de l'exemple 1 sur l'adhésion entre les cellules cutanées.

L'étude est réalisée dans des micro-plaques de 96 puits sur des kératinocytes cultivés dans un incubateur à 37 °C et à 5 % de CO2.

Les puits de ces plaques sont prétraitées, pendant 12 heures, de manière différente ; quatre conditions sont ainsi réalisées :
- Condition A : contrôle négatif, ne contenant pas de peptide ;
- Condition B : incubée avec différents peptides, constitués de 3 à 25 acides aminés (issus de la matrice extra-cellulaire), à une concentration de 5 à 50 ppm ;
- Condition C : incubée avec le peptide de séquence Arg-Asp-Phe-Thr-Lys-Ala-Thr-Asn-Ile-Arg-Leu-Arg-Phe-Leu-Arg à une concentration de 5 ppm.

Après 3 heures de contact avec les kératinocytes, les puits sont totalement remplis de milieu, hermétiquement fermés, retournés et agités sur un agitateur tridimensionnel pendant 20 minutes. Puis les plaques sont vidées et le milieu restant est aspiré. Ensuite, 100 µl de MTT à 1 mg/ml est ajouté par puits et laissé pendant 3 heures à 37 °C et à 5 % de CO2. La solution est enfin retirée et 100 µl de DMSO est ajouté. Une lecture de la DO est faite à 560 nm contre 630 nm.

Les résultats présentent les différentes Densités Optiques (D.O.) obtenues en fonction des différentes conditions. La DO est proportionnelle à la quantité de cellules viables, c'est-à-dire celles qui ont adhéré aux micro-plaques.

| Conditions | A | B | C |
|---|---|---|---|
| D.O. | 0,360 | 0,360 | 0,430 |

Ces résultats démontrent, qu'après seulement 3 heures de contact avec les kératinocytes, le peptide de séquence Asp-Phe-Thr-Lys-Ala-Thr-Asn-Ile-Arg-Leu-Arg-Phe-Leu-Arg apporte une très bonne adhésion cellulaire, bien supérieure à celle des autres peptides testés.

### Exemple 3 - Mise en évidence par immunofluorescence de l'effet du peptide de l'exemple 1 sur l'expression de la fibronectine et sur l'expression du collagène de type III.

Le but de l'étude est de déterminer l'influence du peptide de l'exemple 1 sur la synthèse de fibronectine et sur la synthèse de collagène de type III, par les fibroblastes, par la technique d'immunofluorescence.

L'immunofluorescence est une technique semi-quantitative qui permet d'apprécier le taux de chacune des protéines présentes dans le cytoplasme cellulaire.

Des fibroblastes humains sont ensemencés dans des Labteks puis mis en culture pendant une nuit. Après rinçage avec du tampon HBS, une composition contenant une concentration de 10⁻⁶ M du peptide de séquence Asp-Phe-Thr-Lys-Ala-Thr-Asn-Ile-Arg-Leu-Arg-Phe-Leu-Arg, ou une composition contrôle ne contenant pas de peptide, est ajoutée. Les cellules sont ensuite incubées durant 48 heures. Après élimination des surnageants et rinçage des cultures, les cellules sont fixées avec du paraformaldhéyde pendant 30 minutes à 4°C puis rincées avec du tampon PBS.

200µL d'anticorps anti-fibronectine et/ou d'anticorps anti-collagène III sont alors ajoutés. L'incubation dure 30 minutes à température ambiante. Les surnageants sont éliminés et les cellules sont rincées au PBS. 200µL d'anticorps secondaire, couplé à un marqueur fluorescent (la fluorescéine) est ensuite additionné. Après 30 minutes d'incubation à température ambiante, les surnageants sont éliminés et les cellules sont rincées au PBS.

Les lames sont alors montées puis examinées au microscope à fluorescence inversée. Les quantités de fibronectine et/ou de collagène de type III, synthétisées par les cellules, sont proportionnelles à l'intensité de la fluorescence.

Les résultats obtenus démontrent que l'ajout de peptides dans le milieu de culture des fibroblastes a pour effet d'augmenter la synthèse de fibronectine et/ou la synthèse de collagène de type III par les cellules. Cette stimulation a été observée de manière importante.

En effet, lorsque les fibroblastes sont incubés en présence de la composition contenant le peptide, on assiste au bout de 48 heures à une augmentation de l'intensité de la fluorescence traduisant ainsi une stimulation de la synthèse de la fibronectine et/ou une stimulation de la synthèse du collagène de type III par les fibroblastes.

### Exemple 4 - Mise en évidence par immunofluorescence de l'effet du peptide de l'exemple 1 sur l'expression de la laminine-5 et sur l'expression des intégrines β1.

Le but de l'étude est de déterminer l'influence du peptide de l'exemple 1 sur la synthèse de laminine-5 et sur la synthèse des sous-unités β1 des intégrines, par les kératinocytes, par la technique d'immunofluorescence.

Une étude par la technique d'immunofluorescence, identique à celle de l'exemple 3, a été effectuée sur des kératinocytes humains HaCat, avec des anticorps anti-laminine-5 et/ou avec des anticorps anti-intégrine β1.

Les résultats obtenus démontrent que l'ajout de peptides dans le milieu de culture des kératinocytes a pour effet d'augmenter la synthèse de laminine-5 et/ou la synthèse des intégrines β1 par les cellules. Cette stimulation a été observée de manière importante.

En effet, lorsque les kératinocytes sont incubés en présence de la composition contenant le peptide, on assiste, au bout de 48 heures, à une augmentation de l'intensité de la fluorescence traduisant ainsi une stimulation de la synthèse de la laminine-5 et/ou une stimulation de la synthèse des intégrines β1 par les kératinocytes.

### Exemple 5 - Préparation de compositions

Ces compositions ont été obtenues par simple mélange des différents composants. Les quantités indiquées sont données en pourcentage de poids.

### 1 - Emulsion huile dans eau

| Phase huileuse : | | |
|---|---|---|
| ■ Montanov 68 (Cetearyl Alcohol and Cetearyl Glucoside) | | 5,00 % |
| ■ Huile de Jojoba | | 5,00 % |
| ■ Huile de Vaseline | | 5,00 % |
| ■ Isopropyl Palmitate | | 7,00 % |

| Phase aqueuse : | | |
|---|---|---|
| ■ Glycérine | | 5,00 % |
| ■ Allantoïne | | 0,10 % |
| ■ peptide de l'exemple 1 | | 100 ppm |
| ■ Sepigel 305 (Polyacrylamide, C13-14 Isoparaffin and Laureth-7) | | 0,30 % |
| ■ Conservateur | | 0,50 % |
| ■ Parfum | | 0,50 % |
| ■ Eau | qsp | 100 % |

| 2 - Gel | | |
|---|---|---|
| ■ Carbopol Ultrez 10 (sol. à 2% ) | | 25,00 % |
| ■ Triéthanolamine | | 0,50 % |
| ■ peptide de l'exemple 1 | | 5 ppm |
| ■ Conservateur | | 0,20 % |
| ■ EDTA (séquestrant) | | 0,10 % |
| ■ Parfum | | 0,50 % |
| ■ Eau | qsp | 100 % |

| 3 - Lotion | | |
|---|---|---|
| ■ Mono Propylene Glycol | | 1,00 % |
| ■ Allantoïne | | 0,30 % |
| ■ Glycérine | | 1,00 % |
| ■ Cetiol HE (PEG-7 Glyceryl Cocoate) | | 1,00 % |
| ■ peptide de l'exemple 1 | | 0,5 ppm |
| ■ Conservateur | | 0,20 % |
| ■ Parfum | | 0,50 % |
| ■ Eau | qsp | 100 % |

## Revendications

1. Composition cosmétique et/ou pharmaceutique et/ou dermatologique **caractérisée en ce qu'**elle contient, comme principe actif, une quantité efficace d'au moins un peptide de formule (I) : Arg-Asp-Phe-Thr-Lys-Ala-Thr-Asn-Ile-Arg-Leu-Arg-Phe-Leu-Arg.

2. Composition selon la revendication précédente **caractérisée en ce que** le peptide est choisi parmi les peptides dont au moins un groupement fonctionnel est protégé par un groupement protecteur, ce groupement protecteur étant soit une acylation ou une acétylation de l'extrémité amino-terminale, soit une amidation ou une estérification de l'extrémité carboxy-terminale, soit les deux.

3. Composition selon l'une quelconque des revendications précédentes, **caractérisée en ce que** le peptide est obtenu par synthèse chimique ou enzymatique à partir des acides aminés constitutifs ou de leurs dérivés ; ou est obtenu par hydrolyse ménagée de protéines naturelles ; ou bien est obtenu par biotechnologie.

4. Composition selon l'une quelconque des revendications précédentes **caractérisée en ce que** le peptide est présent dans la composition à une concentration représentant de 0,005 à 500 ppm (p/p) et préférentiellement à une concentration représentant de 0,1 à 50 ppm (p/p).

5. Composition selon l'une quelconque des revendications précédentes, **caractérisée en ce qu'**elle se présente sous la forme d'une composition cosmétique et/ou dermatologique adaptée à l'administration par voie topique cutanée comprenant un milieu cosmétiquement ou pharmaceutiquement acceptable.

6. Composition selon l'une quelconque des revendications précédentes **caractérisée en ce que** le peptide est préalablement solubilisé dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés, les polyols cycliques, ou tout mélange de ces solvants.

7. Composition selon l'une quelconque des revendications précédentes **caractérisée en ce que** le peptide est préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

8. Utilisation d'au moins un composé de formule (I) tel que défini selon l'une quelconque des revendications 1 à 3, comme ingrédient actif dans ou pour la préparation d'une composition cosmétique et/ou dermatologique et/ou pharmaceutique.

9. Utilisation d'au moins un composé de formule (I) tel que défini selon l'une quelconque des revendications 1 à 3, comme ingrédient actif, dans ou pour la préparation d'une composition cosmétique et/ou dermatologique et/ou pharmaceutique ; le composé ou la composition le contenant étant destiné à traiter de manière curative et/ou préventive les phénomènes du vieillissement cutané et/ou afin d'améliorer l'aspect de la peau.

10. Utilisation d'au moins un composé de formule (I) tel que défini selon l'une quelconque des revendications 1 à 3, comme ingrédient actif, dans ou pour la préparation d'une composition cosmétique et/ou dermatologique et/ou pharmaceutique ; le composé ou la composition le contenant étant destiné à augmenter l'expression des protéines de la matrice extra-cellulaire.

11. Utilisation d'au moins un composé de formule (I) tel que défini selon l'une quelconque des revendications 1 à 3, comme ingrédient actif, dans ou pour la préparation d'une composition cosmétique et/ou dermatologique et/ou pharmaceutique ; le composé ou la composition le contenant étant destiné à augmenter l'expression des intégrines.

12. Utilisation d'au moins un composé de formule (I) tel que défini selon l'une quelconque des revendications 1 à 3, comme ingrédient actif, dans ou pour la préparation d'une composition cosmétique et/ou dermatologique et/ou pharmaceutique ; le composé ou la composition le contenant étant destiné à augmenter l'adhésion cellulaire.

13. Utilisation d'au moins un composé de formule (I) tel que défini selon l'une quelconque des revendications 1 à 3, comme ingrédient actif, dans ou pour la préparation d'une composition cosmétique et/ou dermatologique et/ou pharmaceutique ; le composé ou la composition le contenant étant destiné à accélérer la cicatrisation et la régénération tissulaire.

14. Utilisation d'au moins un composé de formule (I) tel que défini selon l'une quelconque des revendications 1 à 3, comme ingrédient actif, dans ou pour la préparation d'une composition cosmétique et/ou dermatologique et/ou pharmaceutique ; le composé ou la composition le contenant étant destiné à renforcer la barrière cutanée et de protéger la peau et/ou les cheveux contre tous types d'agressions extérieures.

## Claims

1. Cosmetic and/or pharmaceutical and/or dermatological composition **characterised in that** it contains, as an active principle, an effective amount of at least one peptide of formula (I): Arg-Asp-Phe-Thr-Lys-Ala-Thr-Asn-Ile-Arg-Leu-Arg-Phe-Leu-Arg.

2. Composition according to the previous claim, **characterised in that** the peptide is chosen from the peptides of which at least one functional group is protected by a protecting group, said protecting group being either an acylation or an acetylation of the amino-terminal end, or an amidation or an esterification of the carboxy-terminal end, or both.

3. Composition according to any one of the previous claims, **characterised in that** the peptide is obtained by chemical or enzymatic synthesis from the constituent amino acids or their derivatives, or by controlled hydrolysis of natural proteins, or by biotechnology.

4. Composition according to any one of the previous claims, **characterised in that** the peptide is present in the composition at a concentration representing 0.005 to 500 ppm (p/p) and preferably at a concentration representing 0.1 to 50 ppm (p/p).

5. Composition according to any one of the previous claims, **characterised in that** it is in the form of a cosmetic and/or dermatological composition suitable for topical skin administration, including a cosmetically- or pharmaceutically-acceptable medium.

6. Composition according to any one of the previous claims, **characterised in that** the peptide is pre-solubilised in one or more cosmetically- or pharmaceutically-acceptable solvents such as water, ethanol, propanol or isopropanol, glycol propylene, glycol butylene, glycol dipropylene, ethoxylated or propoxylated diglycols, cyclic polyols, or any mixture of these solvents.

7. Composition according to any one of the previous claims, **characterised in that** the peptide is pre-solubilised in a cosmetic or pharmaceutical vector such as liposomes, or adsorbed on powdery organic polymers, mineral supports such as talcs and bentonites, and more generally solubilised in, or bound to, any cosmetically- or pharmaceutically-acceptable vector.

8. Use of at least one compound of formula (I) as defined according to any one of claims 1 to 3, as the active ingredient, in or for the preparation of a cosmetically- and/or dermatologically and/or pharmaceutically-acceptable composition.

9. Use of at least one compound of formula (I) as defined according to any one of claims 1 to 3, as the active ingredient, in or for the preparation of a cosmetic and/or dermatologic and/or pharmaceutical composition, the compound or the composition containing it being intended to curatively and/or preventively treat skin ageing phenomena and/or to improve the appearance of the skin.

10. Use of at least one compound of formula (I) as defined according to any one of claims 1 to 3, as the active ingredient, in or for the preparation of a cosmetic and/or dermatological and/or pharmaceutical composition, the compound or the composition containing it being intended to enhance the expression of proteins of the extracellular matrix.

11. Use of at least one compound of formula (I) as defined according to any one of claims 1 to 3, as the active ingredient, in or for the preparation of a cosmetic and/or dermatological and/or pharmaceutical composition, the compound or the composition containing it being intended to enhance the expression of integrins.

12. Use of at least one compound of formula (I) as defined according to any one of claims 1 to 3, as the active ingredient, in or for the preparation of a cosmetic and/or dermatological and/or pharmaceutical composition, the compound or the composition containing it being intended to enhance cell adhesion.

13. Use of at least one compound of formula (I) as defined according to any one of claims 1 to 3, as the active ingredient, in or for the preparation of a cosmetic and/or dermatological and/or pharmaceutical composition, the compound or the composition containing it being intended to accelerate scarring and tissue regeneration.

14. Use of at least one compound of formula (I) as defined according to any one of claims 1 to 3, as the active ingredient, in or for the preparation of a cosmetic and/or dermatological and/or pharmaceutical composition, the compound or the composition containing it being intended to reinforce the skin barrier and protect the skin and/or hair from any type of external damage.

## Patentansprüche

1. Kosmetische und/oder pharmazeutische und/oder dermatologische Zusammensetzung, **dadurch gekennzeichnet, dass** sie als aktives Prinzip eine wirksame Menge mindestens eines Peptids der Formel (I) Arg-Asp-Phe-Thr-Lys-Ala-Thr-Asn-Ile-Arg-Leu-Arg-Phe-Leu-Arg enthält.

2. Zusammensetzung nach vorangehendem Anspruch, **dadurch gekennzeichnet, dass** das Peptid aus Peptiden ausgewählt ist, von denen mindestens eine funktionelle Gruppe von einer Schutzgruppe geschützt ist, wobei diese Schutzgruppe entweder eine Acylierung oder eine Acetylierung des aminoterminalen Endes oder eine Amidierung oder eine Veresterung des carboxyterminalen Endes oder beides ist.

3. Zusammensetzung nach einem der vorangehenden Ansprüche, **dadurch gekennzeichnet, dass** das Peptid durch chemische oder enzymatische Synthese aus konstitutiven Aminosäuren oder ihren Derivaten hergestellt ist; oder durch von natürlichen Proteinen gelenkte Hydrolyse; oder aber durch Biotechnologie.

4. Zusammensetzung nach einem der vorangehenden Ansprüche, **dadurch gekennzeichnet, dass** das Peptid in der Zusammensetzung in einer Konzentration von 0,005 bis 500 ppm (p/p) und vorzugsweise in einer Konzentration von 0,1 bis 50 ppm (p/p) vorhanden ist.

5. Zusammensetzung nach einem der vorangehenden Ansprüche, **dadurch gekennzeichnet, dass** sie sich in Form einer kosmetischen und/oder dermatologischen Zusammensetzung darstellt, die zur Verabreichung auf topischem Weg über die Haut geeignet ist, ein kosmetisch oder pharmazeutisch akzeptables Medium umfassend.

6. Zusammensetzung nach einem der vorangehenden Ansprüche, **dadurch gekennzeichnet, dass** das Peptid zuvor in einem oder mehreren kosmetisch oder pharmazeutisch akzeptablen Lösungsmittel wie Wasser, Ethanol, Propanol oder Isopropanol, Propylenglycol, Butylenglycol, Dipropylenglycol, Ethoxy- oder Propoxydiglycolen, zyklischen Polyolen oder allen Gemischen dieser Lösungsmittel gelöst wird.

7. Zusammensetzung nach einem der vorangehenden Ansprüche, **dadurch gekennzeichnet, dass** das Peptid zuvor in einem kosmetischen oder pharmazeutischen Vektor wie den Liposomen gelöst oder auf pulverförmigen organischen Polymeren, mineralischen Trägern wie Talg und Bentoniten adsorbiert wird und allgemeiner in jedem kosmetisch oder pharmazeutisch akzeptablen Vektor gelöst oder auf ihm fixiert wird.

8. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung.

9. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung, wobei die Verbindung oder die Zusammensetzung, die diese enthält, dazu bestimmt ist, Alterungserscheinungen der Haut kurativ und/oder präventiv zu behandeln und/oder um das Aussehen der Haut zu verbessern.

10. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung, wobei die Verbindung oder die Zusammensetzung, die diese enthält, dazu bestimmt ist, die Expression der Proteine der extrazellulären Matrix zu erhöhen.

11. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung, wobei die Verbindung oder die Zusammensetzung, die diese enthält, dazu bestimmt ist, die Expression der Integrine zu erhöhen.

12. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung, wobei die Verbindung oder die Zusammensetzung, die diese enthält, dazu bestimmt ist, die Zelladhäsion zu erhöhen.

13. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung, wobei die Verbindung oder die Zusammensetzung, die diese enthält, dazu bestimmt ist, die Vernarbung und die Regeneration des Gewebes zu beschleunigen.

14. Verwendung von mindestens einer wie nach einem der Ansprüche 1 bis 3 definierten Formelverbindung (I) als aktiver Inhaltsstoff in der oder für die Zubereitung einer kosmetischen und/oder dermatologischen und/oder pharmazeutischen Zusammensetzung, wobei die Verbindung oder die Zusammensetzung, die diese enthält, dazu bestimmt ist, die Hautbarriere zu stärken und die Haut und/oder das Haar vor allen Arten von Aggressionen von außen zu schützen.
